# EUROPEAN PATENT APPLICATION

(11) **EP 2 019 113 A1**
(43) Date of publication of application: **28.01.2009**
(21) Application number: 07113207.0
(22) Date of filing: 26.07.2007
(51) Int. Cl.: C07H 19/24, A61K 31/7052, A61P 31/12

(54) **New Crystalline Salts of 5-Amino-3-(2',3'-di-O-acetyl-beta-D-ribofuranosyl)-3H-thiazolo[4,5-d]pyrimidin-2-one**

(71) Applicant: Anadys Pharmaceuticals, Inc., San Diego, CA 92121 (US)
(72) Inventor: The designation of the inventor has not yet been filed
(74) Representative: Viering, Jentschura & Partner

(57) **Abstract**

The invention relates to new crystalline salts of 5-amino-3-(2',3'-di-*O*-acetyl-beta-D-ribofuranosyl)-3*H*-thiazolo[4,5-*d*]pyrimidin-2-one, their production and usage, and pharmaceutical preparations containing such salts and crystalline forms.

## Description

The present invention relates to new salts of 5-Amino-3-(2',3'-di-O-acetyl-beta-D-ribofuranosyl)-3*H*-thiazolo[4,5-*d*]pyrimidin-2-one and crystalline forms thereof. Also provided are processes for the preparation thereof, pharmaceutical compositions comprising this salt and crystalline forms thereof and uses thereof in therapeutic treatment of warm-blooded animals, especially humans.

5-Amino-3-(2',3'-di-O-acetyl-beta-D-ribofuranosyl)-3H-thiazolo[4,5-*d*]pyrimidin-2-one can be represented by the following formula 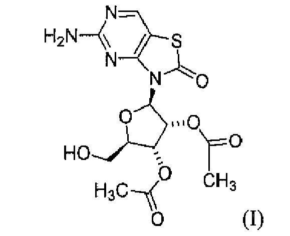 and is known from WO2005/121162, the entire disclosure of which is incorporated by reference, and can be synthesized as described therein. A maleate salt of 5-Amino-3-(2',3'-di-*O*-acetyl-beta-D-ribofuranosyl)-3*H*-thiazolo[4,5-*d*]pyrimidin-2-one and crystalline forms thereof are described in PCT/EP07/054899.

The free base of 5-Amino-3-(2',3'-di-*O*-acetyl-beta-D-ribofuranosyl)-3*H*-thiazolo[4,5-*d*]pyrimidin-2-one is an amorphous substance. It has now been found in accordance with the present invention that salts of 5-Amino-3-(2',3'-di-*O*-acetyl-beta-D-ribofuranosyl)-3*H-*thiazolo[4,5-*d*]pyrimidin-2-one with good crystallinity could be obtained with hydrochloric acid, ethansulfonic acid, gentisic acid and 1-hydroxy-2-naphthoic acid. No degradation of the salts during salt preparation was observed. The new crystalline salts of the present invention show improved physico-chemical properties over the free base in respect to crystallinity, propensity for polymorphism, solubility, stability or hygroscopicity. Consequently, the new crystalline salts of the present invention will provide better physical and chemical stability for us of 5-Amino-3-(2',3'-di-*O*-acetyl-beta-D-ribofuranosyl)-3*H*-thiazolo[4,5-*d*]pyrimidin-2-one as drug substance.

The free base of 5-Amino-3-(2',3'-di-*O*-acetyl-beta-D-ribofuranosyl)-3*H*-thiazolo[4,5-*d*]pyrimidin-2-one is a hygroscopic substance. From the chemical structure it is expected that 5-Amino-3-(2',3'-di-*O*-acetyl-beta-D-ribofuranosyl)-3*H*-thiazolo[4,5-*d*]pyrimidin-2-one is very sensitive to hydrolysis. It has now been found in accordance with the present invention that the new crystalline salts of the present invention are non-hygroscopic or only slightly hygroscopic, in particular the xinafoate, gentisate and hydrochloride salt.

The new crystalline salts of the present invention, in particular the hydrochloride, xinafoate and esylate salt, are highly crystalline, i.e. the salt is largely or virtually all of the salt is in crystralline form and contains only little or virtually no amorphous phase, preferably e.g. less than 1 % (w/w) or than 0.5% (w/w), e.g. at most about 0.1 % (w/w).

The new crystalline salts of the present invention have a good solubility e.g. in an aqueous solvent such as e.g. water or gastric juice. Preferably, the solubility of the crystalline salt of the present invention is at least 0.5 mg/ml, 1 mg/ml, 2 mg/ml, 5 mg/ml or 10 mg/ml in an aqueous solution with low pH such as e.g. a pH between 1 and 6.9.

The free base of 5-Amino-3-(2',3'-di-*O*-acetyl-beta-D-ribofuranosyl)-3*H*-thiazolo[4,5-*d*]pyrimidin-2-one has been found to contain some related substances and shows residual solvents and water. The new crystalline salts of the present invention now enable preparations of 5-Amino-3-(2',3'-di-*O*-acetyl-beta-D-ribofuranosyl)-3*H*-thiazolo[4,5-*d*]pyrimidin-2-one with higher purity, e.g having less related substances and less residual solvents and/or water.

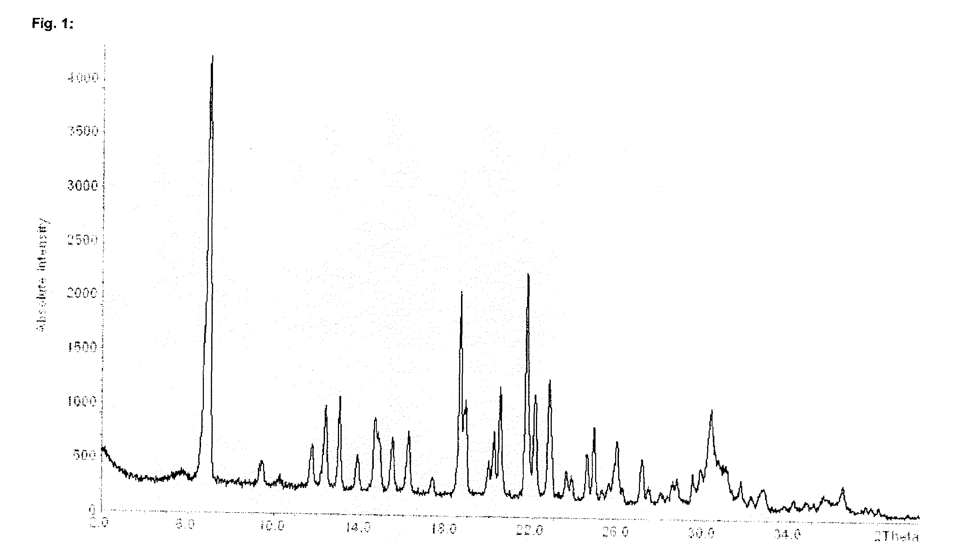

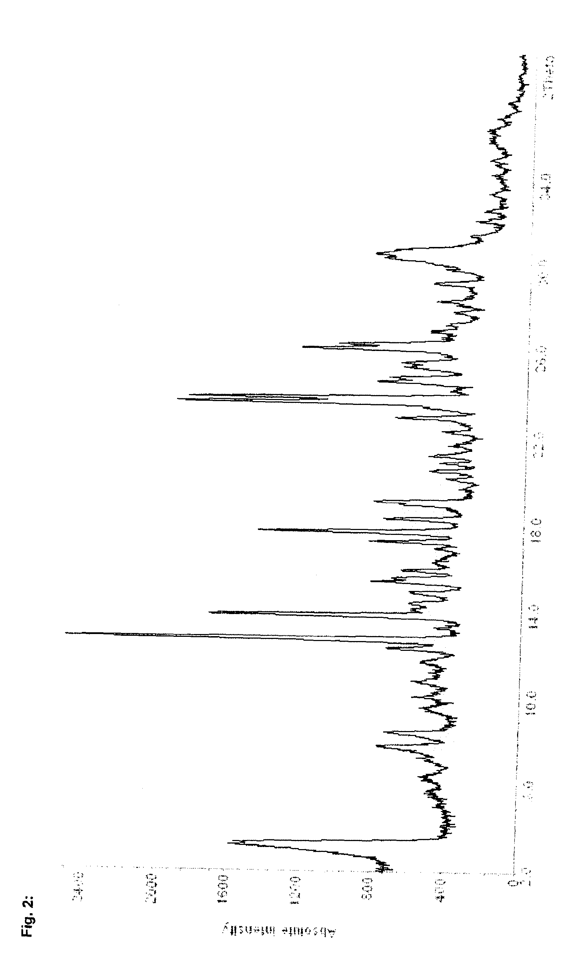

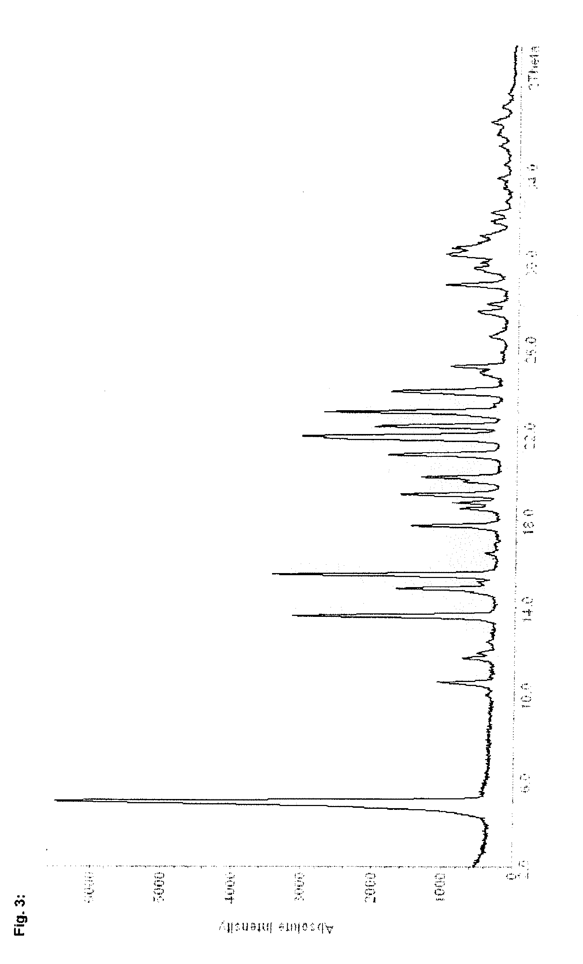

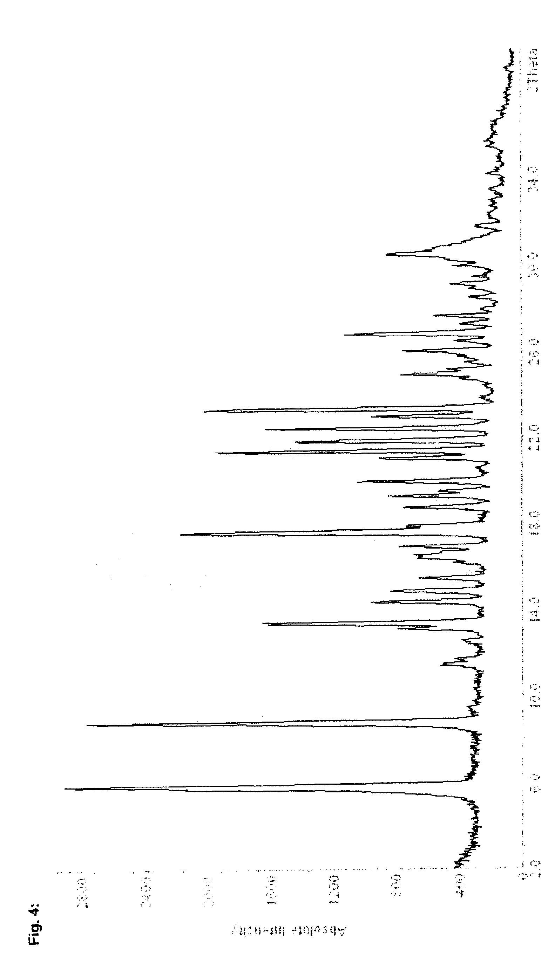

The new crystalline salts of the present invention can e.g. be characterized by X-ray diffraction. Figs. 1, 2, 3 and 4 shows the X-ray diffraction diagram of the new crystalline salts of the present invention. In the X-ray diagram, the angle of diffraction 2theta is plotted on the horizontal axis (x-axis) and the peak intensity on the vertical (*y*-axis). X-ray powder diffraction patterns are measured on a Scintag X1 diffractometer with Cu Kα radiation source (Kα1 radiation, wavelength λ = 1.54060 Angström).

**Table 1: Most significant XRPD reflections (2-theta)**

| **Xinafoate** | | **Gentisate** | | **HCl** | | **Esylate** | |
|---|---|---|---|---|---|---|---|
| **2theta (deg)** | **rel. intensity** | **2theta (deg)** | **rel. intensity** | **2theta (deg)** | **rel. intensity** | **2theta (deg)** | **rel. intensity** |
| 5.0 | strong | 3.2 | medium | 7.0 | strong | 5.9 | strong |
| 13.6 | medium | 12.8 | strong | 12.4 | weak | 8.8 | strong |
| 15.5 | medium | 13.9 | strong | 13.0 | weak | 13.5 | medium |
| 17.7 | weak | 17.8 | medium | 14.7 | weak | 17.7 | strong |
| 19.2 | weak | 23.8 | strong | 15.5 | weak | 21.5 | strong |
| 21.8 | medium | 24.0 | strong | 16.2 | weak | 21.9 | medium |
| 22.4 | medium | | | 18.7 | medium | 23.4 | strong |
| 23.0 | medium | | | 21.7 | medium | 26.9 | strong |
| 24.0 | medium | | | 22.8 | medium | | |

Accordingly, the present invention provides crystalline xinafoate, gentisate, HCl and esylate salt which can be characterized by at least one, preferably two, three, four or more diffractions at angles of diffraction 2theta as set forth in Table 2.

Xinafoate salt:
A characteristic line in the X-ray diffraction diagram can be observed at an angle of diffraction 2theta of 5.0° having a strong intensity. Further characteristic lines can be observed e.g. at 13.6° and 15.5°. More broadly, the crystalline xinafoate salt can be characterized by one or several of diffractions peaks at angles of diffraction 2theta of 5.0°, 13.6°, 15.5° 17.7°, 19.2°, 6.9°, 21.8°, 22.4°, 23.0°, 24.0°.

Gentisate salt:
A characteristic line in the X-ray diffraction diagram can be observed at an angle of diffraction 2theta of 12.8° having a strong intensity. Further characteristic lines can be observed e.g. at 13.9° and 23.8°. More broadly, the crystalline gentisate salt can be characterized by one or several of diffractions peaks at angles of diffraction 2theta of 3.2°, 12.8°, 13.9°, 17.8°, 23.8°, 24.0°.

HCl salt:
A characteristic line in the X-ray diffraction diagram can be observed at an angle of diffraction 2theta of 7.0° having a strong intensity. Further characteristic lines can be observed e.g. at 18.7° and 21.7°. More broadly, the crystalline HCl salt can be characterized by one or several of diffractions peaks at angles of diffraction 2theta of 7.0°, 12.4°, 13.0°, 14.7°, 15.5°, 16.2°, 18.7°, 21.7°, 22.8°.

Esylate salt:
A characteristic line in the X-ray diffraction diagram can be observed at an angle of diffraction 2theta of 5.9° having a strong intensity. Further characteristic lines can be observed e.g. at 8.8°, 13.5° and 17.7°. More broadly, the crystalline esylate salt can be characterized by one or several of diffractions peaks at angles of diffraction 2theta of 5.9°, 8.8°, 13.5°, 17.7°, 21.5°, 21.9°, 23.4°, 26.9°.

In accordance with the present invention, the observed angle of diffraction 2theta can deviate ±0.1°, ±0.2°, ±0.3°, preferably up to ±10% of the above angles of refraction. As appreciated by the person of skill in the art, the relative intensities of the diffractions can vary depending e.g. on the purity of the sample, sample preparation or the instrument used and also, some of the above peaks may not always be detectable.

The crystalline salts of the present invention can also be characterized by melting onset temperatures: xinafoate e.g. about 110°C to 175°C or about 120°C to 165°C, e.g. about 128°C or 158°C, gentisate e.g. of about 100°C to 150°C or about 110°C to 140°C, e.g. about 118°C or 135°C, HCl about 160°C to 190°C or about 165°C to 180°C, e.g. about 173°C, esylate e.g. about 130°C to 170°C or about 140°C to 160°C, e.g. about 152°C. Melting points can be determined by means of a DSC thermogram using a Mettler-Toledo DSC822. DSC ("differential scanning calorimetry") is the technique of dynamic differential calorimetry. Using this technique, the melting temperature of the salts of the present invention can be measured by heating the samples until a thermal, i.e. an endothermic reaction is detected by means of ultrasensitive sensors. The melting points indicated in this text are determined using a Mettler-Toledo DSC822 apparatus, about 1 to 3 mg of each sample being measured in an aluminium crucible with a perforated lid under an atmosphere of nitrogen at a heating rate of 10°C/min (starting at 30°C). As appreciated by the skilled person melting temperatures may differ depending e.g. on the purity of the sample measured. Deviations of for instance ±10°C may not be uncommon.

**Table 2: DSC (10K/min) melting onset temperatures**

| | **Xinafoate** | **Gentisate** | **HCl** | **Esylate** |
|---|---|---|---|---|
| Melting onset (°C) | 127.6 / 157.8 | 117.5 / 135.3 | 173.3 | 152.1 |

In accordance with one aspect, the present invention provides the invention also includes a process for the preparation of a new salt of the invention which comprises reacting the compound of formula I in free base form with an appropriate acid form and recovering from the reaction mixture the resultant salt. The process of the invention may be effected in conventional manner, e. g. by reaction in an appropriate inert solvent such as TBME, methanol, ethanol or isopropanol.

In accordance with another aspect of the invention, a process for the crystallization of a new crystalline salts of the present invention is provided. The precise conditions under which crystals are formed may now be empirically determined and a number of methods are suitable in practice, including the crystallization conditions as described in Examples 1, 2, 3 and 4.

Crystallization-inducing conditions normally involve the use of an appropriate crystallization-inducing solvent, such as t-butylmethylether (TBME), methanol, ethanol, isopropanol, water, Isopropylacetate or MEK (methyl ethyl ketone) or mixtures thereof. Conveniently, the amorphous compound is dissolved in the solvent at a temperature of normally at least 10° C. The solution may be produced by dissolving in a solvent any one or more of amorphous forms of the compound, and solvates thereof, such as hydrates, methanolates, ethanolates, or isopropanolates. Crystals may then be formed by conversion from solution, crystallization taking place at a temperature of between about 0° C. and the boiling point of the solvent. The dissolution and crystallization may be carried out in various conventional ways. For instance, amorphous compound may be dissolved in a solvent or a mixture of solvents in which it is readily soluble at elevated temperatures but in which it is only sparingly soluble at lower temperatures. Dissolution at elevated temperature is followed by cooling during which the desired crystals crystallize out of solution. A cooling and reheating step may be carried out several times, e.g. at least once, at least twice, at least 3x, at least 5x. The cooling and reheating temperatures are e.g. at least 5° C, at least 10° C or at least 15° C. The low temperature of the cooling/heating cycles may e.g. be less than 15° C, less than 10° C, less than 5° C or less than 0° C, whereas the high temperature may e.g. be at least 15 °C, at least 20° C, at least 25°C or at least 30° C.

Mixed solvents comprising a good solvent in which the compound is readily soluble, preferably, in amounts of at least 1% by weight at 30° C, and a poor solvent in which it is more sparingly soluble, preferably in amounts of not more than about 0.01 % by weight at 30° C, may also be employed provided that crystallization from the mixture at a reduced temperature, of normally at least about, 0° C, is possible using the selected solvent mixture.

Alternatively, the difference in solubility of the crystals in different solvents may be used. For example, the amorphous compound may be dissolved in a good solvent in which it is highly soluble such as one in which it is soluble in amounts of at least 1% by weight at about 30° C and the solution subsequently mixed with a poor solvent in which it is more sparingly soluble, such as one in which it is soluble in amounts of not more than about 0.01% by weight at about 30° C. Thus, the solution of the compound in the good solvent may be added to the poor solvent, while maintaining normally a temperature in excess of about 0° C., or the poor solvent may be added to the solution of the compound in the good solvent, again while normally maintaining a temperature in excess of about 0° C. Examples of good solvents may include lower alcohols, such as methanol, ethanol and isopropanol, n-butanol, 3-methylbutanol or acetone , methyl ethyl ketone, acetonitrile, ethylacetate, propylacetate, isopropylacetate. An example of a poor solvent is e.g. water, tert.-butyl-methylether, pentane, hexane, heptane. Preferably, crystallization is effected at a temperature in the range of about 0° C. to about 40° C.

In an alternative embodiment of the process of the invention, solid amorphous compound is suspended at a temperature of normally at least about 0° C. in a solvent in which it is incompletely soluble, preferably only sparingly soluble, at that temperature. A suspension results in which particles of solid are dispersed, and remain incompletely dissolved in the solvent. Preferably the solids are maintained in a state of suspension by agitation e.g. by shaking or stirring. The suspension is kept at a temperature of normally about 0° C. or higher in order to effect a transformation of the starting solids into crystals. The amorphous solid compound suspended in a suitable solvent may be a solvate, e.g. hydrate, methanolate or ethanolate. The amorphous powder may be derived by drying a solvate.

It is possible to add "seeds" of crystalline material (if available) to the solution in order to induce crystallization.

In one aspect the present invention provides pharmaceutical composition comprising an effective amount of a crystalline form of 5-Amino-3-(2',3'-di-*O*-acetyl-beta-D-ribofuranosyl)-3*H*-thiazolo[4,5-*d*]pyrimidin-2-one hydrochloride, xinafoate, gentisate or esylate salt and a suitable carrier.

One embodiment provides methods of preventing or treating infections of a warm-blooded animal, especially a human, by a pathogenic organism comprising administering an effective amount of a new crystalline salts of the present invention. In a preferred embodiment the pathogenic organism is a bacterial, fungal or viral infection disclosed in WO2005/121162, in a preferred embodiment a viral infection caused by adenovirus, cytomegalovirus, hepatitis A virus (HAV), hepatitis B virus (HBV), flaviviruses including Yellow Fever virus and hepatitis C virus (HCV), herpes simplex type 1 and 2, herpes zoster, human herpesvirus 6, human immunodeficiency virus (HIV), human papilloma virus (HPV), influenza A virus, influenza B virus, measles, parainfluenza virus, poliovirus, poxvirus (including smallpox and monkeypod virus), rhinovirus, respiratory syncytial virus (RSV), multiple families of viruses that cause hemorrhagic fevers, including the Arenaviruses (LCM, Junin virus, Machup virus, Guanarito virus, and Lassa Fever), the Bunyaviruses (Hanta viruses and Rift Valley Fever) and Filoviruses ( Ebola and Marburg virus), a range of viral encephalitides including West Nile virus, LaCrosse virus, California Encephalitis virus, Venezuelan Equine Encephalitis virus, Eastern Equine Encephalitis virus, Western Equine Encephalitis virus, Japanese Encephalitis virus, Kysanur Forest virus, and tickborne viruses such as Crimean-Congo Hemorrhagic fever virus. Particularly preferred are HBV and HCV. Another embodiment provides methods of modulating immune cytokine activities of a warm-blooded animal, especially a human, comprising administering an effective amount of a crystalline form of 5-Amino-3-(2',3'-di-*O-*acetyl-beta-D-ribofuranosyl)-3H-thiazolo[4,5-*d*]pyrimidin-2-one hydrochloride, xinafoate, gentisate or esylate salt. Also provided is a crystalline form of 5-Amino-3-(2',3'-di-*O*-acetyl-beta-D-ribofuranosyl)-3*H*-thiazolo[4,5-*d*]pyrimidin-2-one hydrochloride, xinafoate, gentisate or esylate salt for use in medicine. Also provided is the use of a crystalline form of 5-Amino-3-(2',3'-di-*O*-acetyl-beta-D-ribofuranosyl)-3*H*-thiazolo[4,5-*d*]pyrimidin-2-one hydrochloride, xinafoate, gentisate or esylate salt for the manufacture of a medicament for the treatment of an infection by a pathogen, especially a virus, e.g. HCV or HBV. Further provided is the use of a crystalline form of 5-Amino-3-(2',3'-di-*O*-acetyl-beta-D-ribofuranosyl)-3*H*-thiazolo[4,5-*d*]pyrimidin-2-one hydrochloride, xinafoate, gentisate or esylate salt for the manufacture of a medicament modulating immune cytokine activities of a warm-blooded animal.

The present invention further includes:
- a pharmaceutical composition comprising a salt or a crystalline salt of the invention together with at least one pharmaceutically acceptable carrier or diluent;
- a pharmaceutical composition comprising the compound of formula I in free form or pharmaceutically acceptable salt form other than a hydrochloric acid, ethansulfonic acid, gentisic acid or 1-hydroxy-2-naphthoic acid addition salt form, whenever prepared from a salt or a crystalline salt of the invention;
- a salt or a crystalline salt of the invention for use as a pharmaceutical;
- a salt or a crystalline salt of the invention for use in the preparation of a medicament;
- a salt or a crystalline salt of the invention whenever prepared by a process as defined above;
- a salt or a crystalline salt of formula I in free base form or salt form other than a hydrochloric acid, ethansulfonic acid, gentisic acid or 1-hydroxy-2-naphthoic acid addition salt form, whenever prepared from a salt or a crystalline salt of the invention;
- the use of a compound of the invention in the preparation of a medicament for the treatment, e.g. orally or intravenously, of diseases susceptible of therapy with the salt or the crystalline salt of formula I in free base form or salt form, such as viral diseases;
- a process for the preparation of a pharmaceutical composition which comprises mixing a salt or a crystalline salt of the invention together with at least one pharmaceutically acceptable carrier or diluent; and
- a method for the prophylactic or curative treatment of viral diseases such as HCV or HBV infection, comprising administration of a therapeutically effective amount of a salt or a crystalline salt of the invention to a subject in need of such treatment.

The crystalline forms of the present invention are synthesized in accordance with the following examples which are illustrative without limiting the scope of the present invention.

### Brief Description of the Drawings:

Fig. 1 shows the X-ray diffraction diagram of crystalline hydrochloride salt of 5-Amino-3-(2',3'-di-*O*-acetyl-beta-D-ribofuranosyl)-3*H*-thiazolo[4,5-*d*]pyrimidin-2-one.
Fig. 2 shows the X-ray diffraction diagram of crystalline gentisate salt of 5-Amino-3-(2',3'-di-*O*-acetyl-beta-D-ribofuranosyl)-3*H*-thiazolo[4,5-*d*]pyrimidin-2-one.
Fig. 3 shows the X-ray diffraction diagram of crystalline xinafoate salt of 5-Amino-3-(2',3'-di-*O*-acetyl-beta-D-ribofuranosyl)-3H-thiazolo[4,5-*d*]pyrimidin-2-one.
Fig. 4 shows the X-ray diffraction diagram of crystalline esylate salt of 5-Amino-3-(2',3'-di-*O-*acetyl-beta-D-ribofuranosyl)-3*H*-thiazolo[4,5-*d*]pyrimidin-2-one,

### Example 1: 5-Amino-3-(2',3'-di-O-acetyl-beta-D-ribofuranosyl)-3H-thiazolo[4,5-d]pyrimidin-2-one Hydrochloride

5.0 g 5-Amino-3-(2',3'-di-*O*-acetyl-beta-D-ribofuranosyl)-3*H*-thiazolo[4,5-*d*]pyrimidin-2-one base (13.01 mmoles) are dissolved in 15 ml ethanol at 50°C. 8.35 g of a hydrochloric acid 5.45 wt. % solution in ethanol (12.36 mmoles) are then added dropwise over ca. 10 min. The dropping funnel is rinsed with 1 ml ethanol. The solution is clarified via filtration at 50 °C over a glasfiber filter. The filter is rinsed with 5 ml ethanol of 50°C. The filtrate is allowed to cool. Crystallization takes place after seeding at 35°C. The suspension is cooled to 0°C over ca. 2 hours and stirred over night at this temperature. The slurry is filtrated and the filter cake washed with 8 ml cold ethanol. The crystals are dried at 60 °C and ca. 8 mbar for ca. 20 h. Yield: 4.96 g white powder (95.37 %).

### Example 2: 5-Amino-3-(2',3'-di-O-acetyl-beta-D-ribofuranosyl)-3H-thiazolo[4,5-d]pyrimidin-2-one Esylate

5.0 g 5-Amino-3-(2',3'-di-*O*-acetyl-beta-D-ribofuranosyl)-3*H*-thiazolo[4,5-*d*]pyrimidin-2-one base (13.01 mmoles) are dissolved in 15 ml methyl-ethylketone (MEK) and the solution is heated to 50°C. A solution of 1.43 g ethansulfonic acid (12.36 mmoles) in 3 ml MEK is added dropwise over ca. 5 min. The dropping funnel is rinsed with 2 ml MEK. The solution is clarified via filtration at 50 °C over a glasfiber filter. The filter is rinsed with 5 ml MEK of 50°C. The filtrate is allowed to cool. Some seeds are added at 30°C and crystallization takes place. The suspension is stirred at room temperature over night and then further stirred at 0°C for two hours. The solid is collected after filtration and washing with 6 ml MEK of 0°C. The salt is dried at 60°C and 8 mbar for ca. 20 h.
Yield: 5.70 g white powder (93.27%)

### Example 3: 5-Amino-3-(2',3'-di-O-acetyl-bete-D-ribofuranosyl)-3H-thiazolo[4,5-d]pyrimidin-2-one Gentisate

5.0 g 5-Amino-3-(2',3'-di-O-acetyi-beta-D-ribofuranosyl)-3*H*-thiazolo[4,5-*d*]pyrimidin-2-one base (13.01 mmoles) are dissolved in 25 ml isopropylacetate at 50°C. 1.94 g 2,5-dihydroxybenzoic (12.36 mmoles) acid are added. the resulting solution is clarified via filtration at 50°C over a glasfiber filter. The filter is rinsed with 5 ml isopropylacetate of 50°C. The clear filtrate is allowed to cool. Crystallization takes place after seeding at 35°C. The slurry becomes rapidly too thick and is gradually diluted with 10 ml isopropylacetate. The suspension is cooled to 0°C over ca. 2 hours and stirred at this temperature over night. The slurry is filtered and the filter cake is washed with 6 ml isopropylacetate of 0°C. The solid is dried at 50°C and ca. 10 mbar for 20 h.
Yield: 5.35 g slighty yellowish powder (80.4 %)

### Example 4: 5-Amino-3-(2',3'-di-O-acetyl-beta-D-ribofuranosyl)-3H-thiazolo[4,5-d]pyrimidin-2-one Xinafoate

A suspension of 5.0 g 5-Amino-3-(2',3'-di-*O*-acetyl-beta-D-ribofuranosyl)-3*H*-thiazolo[4,5-*d*]pyrimidin-2-one base (13.01 mmoles) and 2.37 g 1-hydroxy-2-naphthoic acid (12.36 mmoles) in 25 ml isopropylacetate is heated to 50°C. The resulting cloudy solution is clarified via filtration at 50°C over a glasfiber filter. The filter is rinsed with 5 ml isopropylacetate of 50°C. The clear filtrate is allowed to cool. Crystallization takes place after seeding at 30°C. The slurry becomes rapidly too thick and is gradually diluted with 10 ml isopropylacetate. The suspension is cooled to 0°C over ca. 2 hours and stirred at this temperature over night. The slurry is filtered and the filter cake is washed with 6 ml isopropylacetate of 0°C. The solid is dried at 50°C and ca. 10 mbar for 20 h.
Yield: 5.82 g white powder (82.26%)

**Table 3:**

| | **Salt** | **Rel. Mol. mass** | **Solvent of crystallization** | **Yield (%)** | **Purity by HPLC (%)** |
|---|---|---|---|---|---|
| 1 | Base | 384.36 | | | 95.01 |
| 2 | Xinafoate | 572.55 | Isopropylacetate | 82.26 | 98.25 |
| 3 | Gentisate | 538.49 | Isopropylacetate | 80.40 | 98.25 |
| 4 | Hydrochlorid e | 420.83 | Ethanol | 95.37 | 98.85 |
| 6 | Esylate | 495.50 | MEK | 93.27 | 98.85 |

| | | | | | |
|---|---|---|---|---|---|
| Good purification effect by salt formation observed in all four cases. | | | | | |

### Example 5: Methodology, Instruments and Standards that can be used to characterize the crystalline salts of the present invention

| | | |
|---|---|---|
| 1 | Determination of approximate solubility To 10 mg DS solvent was added in portions (0.5; 0.5; 1.0; 2.0; 6.0 ml). The solubility was estimated according to the total volume of added solvent. | |
| 2 | Hygroscopicity Water sorption curves were recorded using a DVS-1 system at 25°C and rel. humidities 0-95%r.h. Samples werer also stored for 24hrs at 80%r.h. and 92%r.h. | |
| 3 | Equilibration studies were performed using a Mettler Multiblock. About 75mg of drug substance were suspended in about 2ml of solvent. Equilibration were performed at 25°C for about 24h. The suspension were filtrated and the reaming solid analysed by XRPD. The filtrate was allowed to evaporate under ambient conditions and remaining solids were also analysed by XRPD. | |
| 4 | DSC method | |
| | Instrument | Mettler DSC822e |
| | Temperature range | 30°-300°C |
| | Scan rate | 1-20K/min |
| 5 | XRPD method | |
| | Instrument (transmission) | STOE, Powder Diffraction System |
| | Irradiation | CuKα (50 kV, 30 mA) |
| | Chopper | 0.02 grd |
| | Scan rate | 0.5sec/step |
| | Scan range | 2° - 40° (2 theta value) |
| | Instrument (reflection) | Bruker D8 Advanced, Bruker |
| | Irradiation | CuKα (30 kV, 40 mA) |
| | Chopper | 0.02 grd |
| | Scan type | Continuous scan |
| | Scan rate | 3sec/step |
| | Scan range | 2° - 40° (2 theta value) |

## Claims

1. A hydrochloride, gentisate, xinafoate or esylate salt of 5-Amino-3-(2',3'-di-*O*-acetyl-beta-D-ribofuranosyl)-3*H*-thiazolo[4,5-*d*]pyrimidin-2-one.

2. A hydrochloride, gentisate, xinafoate or esylate salt of 5-Amino-3-(2',3'-di-*O*-acetyl-beta-D-ribofuranosyl)-3*H*-thiazoio[4,5-*d*]pyrimidin-2-one in crystalline form.

3. A crystalline salt according to claim 2 which shows on X-ray diffraction a peak at an angle of refraction 2theta of 7.0°±0.3° (HCl), 12.8°±0.3° (Gentisate), 5.0°±0.3° (Xinafoate), 12.8°±0.3° (Gentisate), or 5.9°±0.3° (Esylate).

4. A crystalline salt according to claim 3 which further shows on X-ray diffraction a peak at an angle of refraction 2theta of 18.7°±0.3° and 21.7°±0.3° (HCl), 13.9°±0.3° and 23.8°±0.3° (Gentisate), 13.6°±0.3° and 15.5°±0.3° (Xinafoate) or 8.8°±0.3°, 13.5°±0.3° and 17.7°±0.3° (Esylate).

5. A crystalline salt according to claim 2, 3 or 4 which comprises at least three of the peaks as defined in Table 1.

6. A crystalline salt according to claim 2, 3, 4 or 5 which comprises at least three of the peaks displayed in Figure 1, Figure 2, Figure 3 or Figure 4.

7. A salt or crystalline salt according to claim 1 to 6 which is present in essentially pure form.

8. A crystalline salt according to claim 2 to 7 having a high crystallinity.

9. A process for the preparation of a hydrochloride, gentisate, xinafoate or esylate salt of 5-amino-3-(2',3'-di-*O*-acetyl-beta-D-ribofuranosyl)-3*H*-thiazolo[4,5-*d*]pyrimidin-2-one according to claim 1 comprising reacting 5-amino-3-(2',3'-di-*O*-acetyl-beta-D-ribofuranosyl)-3*H*-thiazolo[4,5-*d*]pyrimidin-2-one with hydrochloric acid, ethansulfonic acid, gentisic acid or 1-hydroxy-2-naphthoic acid and recovering from the reaction mixture the resultant salt.

10. A process for the preparation of a crystalline hydrochloride, gentisate, xinafoate or esylate salt salt of 5-amino-3-(2',3'-di-*O*-acetyl-beta-D-ribofuranosyl)-3*H*-thiazolo[4,5-*d*]pyrimidin-2-one according to claim 2 to 7 comprising appropriately converting amorphous hydrochloride, gentisate, xinafoate or esylate salt of 5-Amino-3-(2',3'-di-*O-*acetyl-beta-D-ribofuranosyl)-3*H*-thiazolo[4,5-*d*]pyrimidin-2-one from a solution thereof under crystallization-inducing conditions.

11. A process for the preparation of a crystalline hydrochloride, gentisate, xinafoate or esylate salt of 5-amino-3-(2',3'-di-*O*-acetyl-beta-D-ribofuranosyl)-3*H*-thiazolo[4,5-*d*]pyrimidin-2-one according to claim 2 to 8 comprising the step of crystallization or recrystallization a hydrochloride, gentisate, xinafoate or esylate salt of 5-amino-3-(2',3'-di-*O*-acetyl-beta-D-ribofuranosyl)-3*H*-thiazolo[4,5-*d*]pyrimidin-2-one in a solution comprising ethanol, isopropylacetate or MEK.

12. A pharmaceutical composition comprising a hydrochloride, gentisate, xinafoate or esylate salt or a crystalline form of a hydrochloride, gentisate, xinafoate or esylate salt according to claim 1 to 8.

13. A method of treating an infection by a pathogen, comprising administering to a patient in need a therapeutically effective amount of a hydrochloride, gentisate, xinafoate or esylate salt or a crystalline form of a hydrochloride, gentisate, xinafoate or esylate salt according to claim 1 to 8.

14. A hydrochloride, gentisate, xinafoate or esylate salt or a crystalline form of a hydrochloride, gentisate, xinafoate or esylate salt according to claim 1 to 8 for use in medicine.

15. Use of a hydrochloride, gentisate, xinafoate or esylate salt or crystalline form of a hydrochloride, gentisate, xinafoate or esylate salt according to claim 1 to 8 for the manufacture of a medicament for the treatment of an infection by a pathogen.

16. The use of claim 16 wherein the pathogen is a virus an in particular HCV or HBV.
